# EUROPEAN PATENT SPECIFICATION

(11) **EP 2 076 165 B1**
(45) Date of publication and mention of the grant of the patent: **24.04.2013**
(21) Application number: 07841695.5
(22) Date of filing: 31.08.2007
(51) Int. Cl.: A61B 1/04

(54) **FIBERSCOPE INTERFACING APPARATUS FOR ENDOSCOPIC PROCEDURES**
FIBROSKOP-SCHNITTSTELLENVORRICHTUNG FÜR ENDOSKOPISCHE VERFAHREN
APPAREIL D'INTERFAÇAGE DE FIBERSCOPE POUR DES INTERVENTIONS ENDOSCOPIQUES

(30) Priority: 31.08.2006 US 841593 P
(43) Date of publication of application: 08.07.2009
(73) Proprietor: Cook Medical Technologies LLC, Bloomington, IN 47404 (US)
(72) Inventor: JONES, Brian, K., Spartanburg, SC 29306 (US)
(74) Representative: Garratt, Peter Douglas
(86) International application number: PCT/US2007/077350
(87) International publication number: WO 2008/028107

(56) References cited:
- DE-A1- 10 136 145
- JP-A- 8 227 040
- US-A- 5 196 928
- US-A1- 2003 025 830

## Description

### CROSS-REFERENCE TO RELATED-APPLICATIONS

This application claims the benefit of U.S Provisional Application serial no. 60/841,593 filed on August 31, 2006, entitled "FIBERSCOPE INTERFACING APPARATUS FOR ENDOSCOPIC PROCEDURES.

### FIELD OF THE INVENTION

The present invention relates to medical devices and more particularly to fiberscope interfacing apparatus for endoscopic procedures.

### BACKGROUND OF THE INVENTION

There have been recent advancements of minimally-invasive surgical procedures involving endoscopes. Such procedures have proven to be advantageous alternatives over prior invasive surgical procedures. Such advantages include quicker recovery time as well as more efficient hospital stays and medical costs.

Endoscopic devices have been commonly used for various procedures, typically in the abdominal area. Endoscopy is the examination and inspection of the interior of body organs, joints or cavities: through an endoscope. Endoscopy allows physicians to peer through the body's passageways. An endoscopic procedure may be used to diagnose various conditions by close examination of internal organ and body structures and may also guide therapy and repair, such as the removal of tom cartilage from the bearing surfaces of a joint. A biopsy, a procedure involving tissue sampling for pathologic testing, may also be performed under endoscopic guidance. For example, endoscopic procedures include the following known procedures: gastroscopy, sigmoidoscopy and colonoscopy, esophago gastro duodenoscopy (EGD), endoscopic retrograde cholangiopancreatography (ERCP), and bronchoscopy.

Although adequate, many devices and procedures may be enhanced. For example, there is a need to enhance viewing and viewing efficiency during fluoroscopic and endoscopic procedures. Typically, fluoroscopy is performed separate from an endoscopic procedure. The series of procedures or diagnosis results In extra time involved to complete the diagnosis. Thus, there is a need to enhance imaging features within a body cavity or vessel during endoscopic and fluoroscopic procedures.

### BRIEF SUMMARY OF THE INVENTION

The present invention generally provides a way of enhancing imaging analysis of the internal structures of a patient during an endoscopic and fluoroscopic procedure.
The scope of the present invention is set forth in the appended claims.

One embodiment comprises an endoscopic apparatus having simultaneous fluoroscopic and fiber optic imaging features for a body cavity. The endoscopic apparatus comprises an endoscope comprising a control system for controlling the endoscope, an insertable tube extending from the control system to a distal tip wherein the insertable tube is in communication with the control system, and a scope medium extending to a scope connector in communication with the control system. The endoscopic apparatus further comprises a system tower configured to receive the scope connector for communication with the control system and a fiberscope interfacing apparatus. The fiberscope interfacing apparatus is cooperable with the endoscope. The fiberscope interfacing apparatus comprises an elongate communication medium having a first end and a second end for transmitting an optical signal of an image of the body cavity, a lens attached to the first end of the communication medium for receiving the optical signal from the body cavity, the lens being in communication with the communication medium and the fiberscope interfacing apparatus further comprises an interface collar attached to the second end of the communication medium and in communication therewith and configured to connect to the scope connector for receiving an endoscope digital image signal. The interface collar is configured to convert a fiberscope optical signal to a fiberscope digital signal. The interface collar is further configured to convert the fiberscope digital image signal and the endoscope digital signal to a combined digital image signal.. The endoscopic apparatus further comprises a video imaging apparatus that is configured to connect to the interface collar for receiving the combined digital image signal for simultaneous fluoroscopic and fiber optic Imaging.

In another example, there is provided a method of simultaneous fluoroscopic and fiber optic imaging during an endoscopic procedure of a body cavity. The method comprises receiving a fiber optic image signal and converting the fiber optic image signal to a fiberscope digital signal. The method further comprises combining fiberscope digital signal with a fluoroscopic digital image signal to define a combined digital image signal. The method further comprises displaying the combined digital image signal for simultaneous imaging of the body cavity.

Further objects, features, and advantages of the present invention will become apparent from consideration of the following description and the appended claims when taken in connection with the accompanying drawings.

### BRIEF DESCRIPTION OF THE DRAWINGS

Figure 1a is a perspective view of an endoscopic system having simultaneous fluoroscopic and fiber optic imaging features in accordance with one embodiment of the present invention;

Figure 1b is an elevated view of the endoscope system of Figure 1a;

Figure 1c is a perspective view of an elongate communication medium of a fiberscope interfacing apparatus in accordance with one embodiment of the present invention;

Figure 1d is a side view of the fiberscope interfacing apparatus in accordance with one embodiment of the present invention;

Figure 2 is a schematic view of the endoscope system implementing the fiberscope interfacing apparatus for simultaneous fiber optic imaging and fluoroscopic imaging;

Figure 3 is a side view of an interface collar for simultaneous fiber optic imaging and fluoroscopic imaging in accordance with one embodiment of the present invention;

Figure 4 is a flow chart of one method of simultaneous fiber optic and fluoroscopic imaging for an endoscope system in accordance with one example; and

Figure 5 is a perspective view of a fluoroscope that may be used in combination with the fiberscope interfacing apparatus of the present invention.

### DETAILED DESCRIPTION OF THE INVENTION

The present invention generally provides a fiberscope interfacing apparatus for an endoscope system having the capability of simultaneous fiber optic imaging and fluoroscopic imaging. This is accomplished by integrating an interface collar of the fiberscope interfacing apparatus with a video system of the endoscope system. The interface collar is configured to receive endoscope digital images and fiberscope images, combines the images for simultaneous viewing such as in a "picture-in-picture" signal, and transmits the images to the video system.

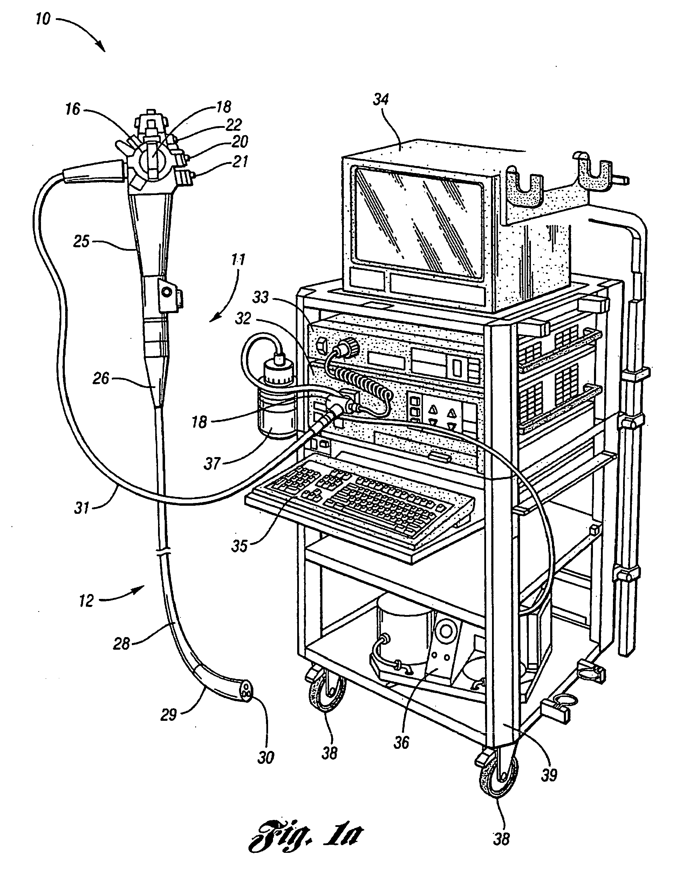

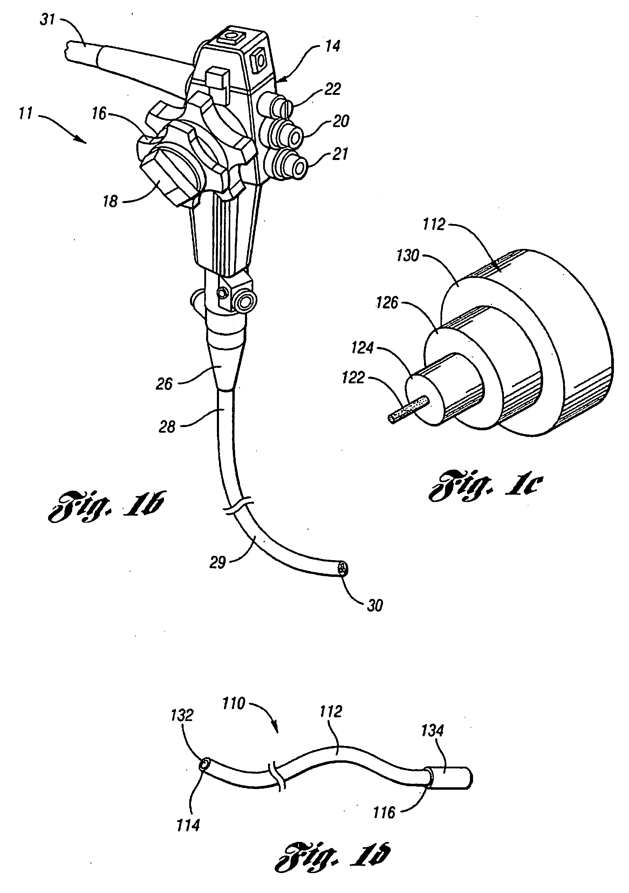

Figure 1a illustrates an endoscopic system 10 comprising an endoscope 11 having an insertion tube 12 in accordance with one embodiment of the present invention. In this embodiment, the insertion tube 12 is configured to be inserted into a body cavity for various endoscopic procedures including gastroscopy, sigmoidoscopy and colonoscopy, esophago gastro duodenoscopy (EGD), endoscopic retrograde cholangiopancreatography (ERCP), and bronchoscopy. As shown, the system 10 further comprises a fiberscope interfacing apparatus 110. In this embodiment, the insertion tube 12 has a plurality of channel ports through which endoscopic units may be disposed, e.g., the fiberscope interfacing apparatus 110 (see Figure 1b and 1d).

As shown in Figures 1a and 1b, the endoscope 11 includes a control system 14 that is in mechanical and fluid communication with the insertion tube 12. The control system 14 is configured to control the insertion tube 12 and endoscopic parts disposed therein. As shown, the control system 14 includes first and second control knobs 16, 18. The control knobs 16, 18 are configured to be in mechanical communication with the insertion tube 12. The control knobs 16, 18 allow the clinician to control and guide, by known means, the insertion tube 12 through vessels and cavities of a patient. The control system 14 further includes valve switches (e.g., suction valve 20, air/water valve 21, fluoroscopic/camera valve 22), each of which are in communication to one of the channel ports of the insertion tube 12. For example, the suction valve switch 20, when activated, allows a vacuum from a suction source through a suction channel port for suctioning unwanted plaque and debris from the patient. In one example, the distal end of the insertion tube 12 is inserted, rectally or orally, to a predetermined endoscopic location within a patient. Insertion of the insertion tube 12 may be rectally or orally depending on the endoscopic procedure.

In this embodiment, the insertion tube 12 comprises an operating portion 25 connected to the control system 14 and extending to an insertion portion protecting member 26. The control system 14 is connected to the operating portion 25 and is configured to control the insertion tube 12. In this embodiment, the insertion tube 12 is composed of components that include a flexible tube 28, a flexure 29 connected to the flexible tube 28, and an endoscope tip 30 connect to the flexure 29. A scope medium or a universal cord 31, on one end, is connected and in communication with the control system 14. On the other end, the cord 31 has a connector 18 attached thereto. The connector 18 is in communication with a light guide tube and electrical contact, and is connected to an endoscopy system tower or a scope tower 32 including a light source apparatus and a video imaging apparatus (video tower) or an image processing apparatus 33. These external devices may include a monitor 34, an input keyboard 35, a suction pump apparatus 36, and an irrigation bottle 37, and other suitable apparatus are installed on a rack 39 equipped with rollers 38.

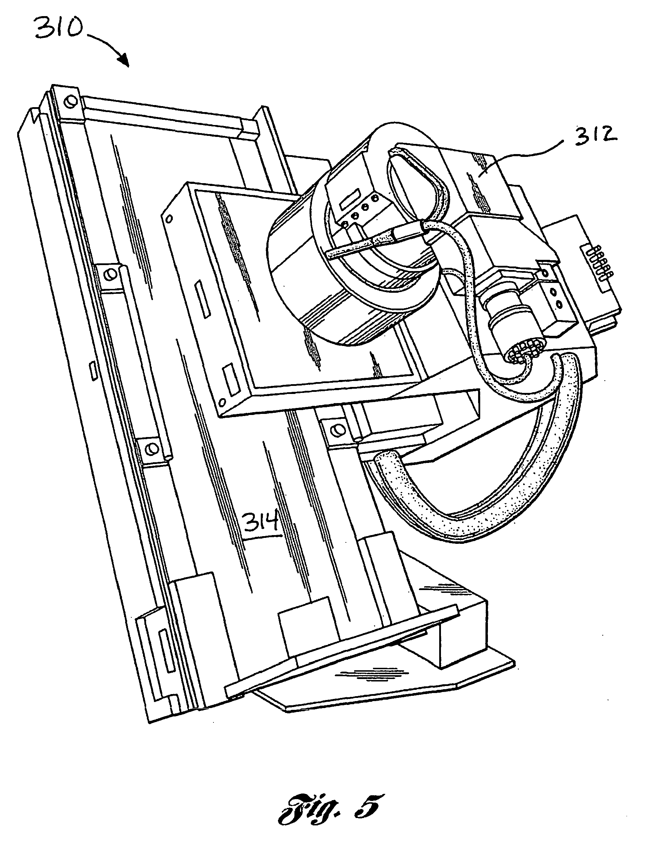

In one example, the distal end of the insertion tube 12 is inserted to a predetermined endoscopic location within a patient. Insertion of the insertion tube 12 may be rectally or orally depending on the endoscopic procedure. At the location, a physician may activate and control the endoscopic units as desired. The endoscope in combination with a fluoroscope and the fiberscope interfacing apparatus of the present invention allows for simultaneous fluoroscopic and fiber optic imaging. For example, this may be accomplished by injecting fluoroscopic dye via a cannula (not shown) through the fluoroscopic valve 22, through the endoscope tip 30, and to a body vessel/cavity location in a patient. By a fluoroscope (Figure 5), fluoroscopy may be conducted to receive real-time images of internal structures through which an endoscope may otherwise not be able to pass, e.g., parts of the biliary tree of the patient.

Fluoroscopy is a study of moving body structures and is a procedure that provides motion video of an x-ray. Fluoroscopy enables physicians and other clinicians to view many body systems, including the skeletal, digestive, urinary, respiratory, and reproductive systems. Fluoroscopy may be performed to evaluate specific areas of the body, including the bones, muscles, and joints, as well as solid organs such as the heart, lung, or kidneys.

In this embodiment, the fluoroscope comprises an x-ray machine and a fluorescent screen (in communication with the x-ray machine) that may be used by physicians and other clinicians to view the internal organs of the body. During medical diagnosis, the patient is placed between the x-ray machine and the fluorescent screen. A continuous x-ray beam is passed through a body part being examined and is transmitted by a scanner to the video imaging apparatus 33 so that the body part and its motion can be seen in detail. As radiation passes through the body, varying degrees of light and shadow on the screen is produced.

Fluoroscopy may be used in various types of examinations and procedures, such as barium x-rays, cardiac catheterization, arthrography (visualization of a joint or joints), lumbar puncture, placement of intravenous catheters, intravenous pyelogram, hysterosalpingogram, and biopsies. For example, in barium x-rays, fluoroscopy allows the physician to see the movement of the intestines as the barium moves through them. In cardiac catheterization, fluoroscopy is added to enable the physician to see the flow of blood through the coronary arteries in order to evaluate the presence of arterial blockages. For intravenous catheter insertion, fluoroscopy assists the physician in guiding the catheter into a specific location inside the body.

During the same procedure and at the same time, the fiberscope interfacing apparatus (discussed in greater detail below) may be used with the endoscope to receive real-time digital images of internal structures through which the endoscope is introduced, e.g., the duodenum or the small intestine of the patient. The signals or images from each of the fluoroscope and fiberscope interfacing apparatus may then be combined and displayed through a video monitor as cascading views, picture-in-picture, alternating views, or any other suitable views.

Figures 1b-2 illustrate a fiberscope interfacing apparatus 110 for the endoscope system 10 in accordance with one embodiment of the present invention. As shown, the fiberscope interlacing apparatus 110 comprises an elongate communication tube or medium 112 for transmitting a light signal or an optical signal of an image from the body cavity. In this embodiment, the elongate communication medium 112 has a first (or distal) end 114 and a second (or proximal) end 115 through which an optical signal may be transmitted by the endoscope to the body cavity and then to an imaging system as described in greater detail below.

In this embodiment, the elongate communication tube 112 is an optical fiber medium that includes at least one fiber core 122 having a predetermined refractive index. Preferably, an optical fiber is a cylindrical dielectric waveguide that transmits light along its longitudinal axis by the process of total internal reflection. In this embodiment, the medium 112 further comprises a cladding layer 124 disposed about each of the fiber cores 122 for transmission of optical signals from the first end 114 to the second end 115 of the communication medium 112. Preferably, each cladding layer 124 has a refractive index that is less than the predetermined refractive index of each of the fiber cores. That is, for total internal reflection to confine the optical signal in the core, the refractive index of the core 122 is preferably greater than that of the cladding layer 124.

Preferably, each cladding layer 124 is coated with a resin buffer layer 126. The resin buffer layer 126 may be made on any suitable non-conductive material such a polymeric material. In this embodiment, the medium 112 further comprises a polymeric jacket layer 130 disposed about one or all of the resin buffer layers 126 for added strength to the fiber core 122.

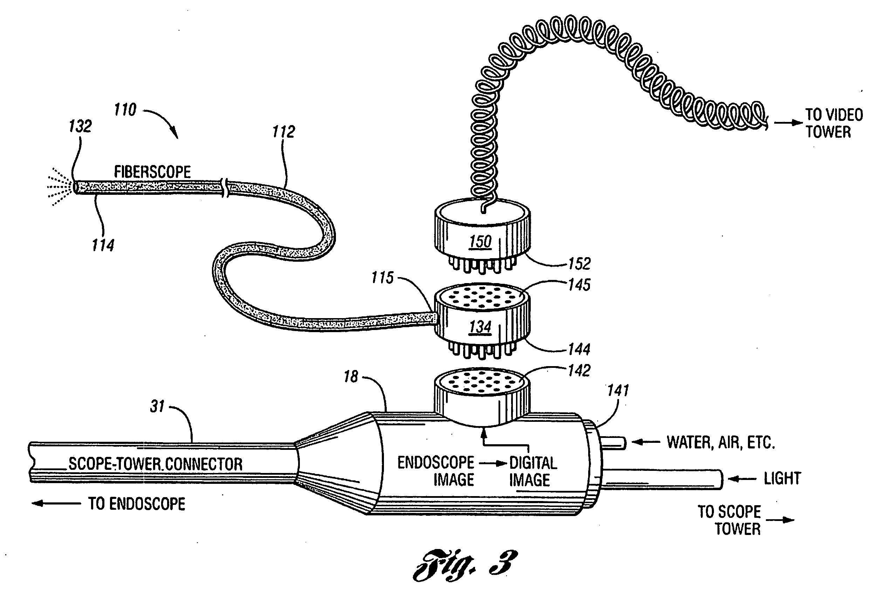

In this embodiment, the fiberscope interfacing apparatus 110 further comprises a lens 132 (Figures 1d and 3) attached to the first (or distal) end 114 of the communication medium 112 by any suitable means. Preferably, the lens 132 is configured to receive optical signals transmitted by the endoscope through the body cavity and is in communication with the communication medium 112 to allow transmission of optical signals therethrough. The lens 132 may be any suitable type of lens, e.g., a wide-angle lens, for receiving optical signals therethrough. In use, light transmitted by through the endoscope to the body cavity is preferably provided by the light source apparatus of the system tower 32 via the scope medium 31 and insertion tube 12.

As shown, the fiberscope interfacing apparatus 110 further comprises an interface collar 134 attached to and in communication with the second (or proximal) end 115 of the communication medium 112. Preferably, the interface collar 134 is configured to communicate with the external devices, e.g., the system tower 32 and the video imaging apparatus 33 of the endoscope system 10 to transmit light or optical signals thereto for simultaneous fiber optic and fluoroscopic imaging as the system tower 32 of the endoscopic system 10 is in communication with the control system 14.

As mentioned above, the scope medium 31 extends to a scope connector 18. The interface collar 134 attaches to the connector 18, generally receives the endoscope digital signal, and converts the fiberscope image signal to a fiberscope digital signal. The interface collar 134 combines the endoscope digital signal and the fluoroscopic image signal into a combined signal for simultaneous imaging, e.g., a cascading view or a picture-in-picture view.

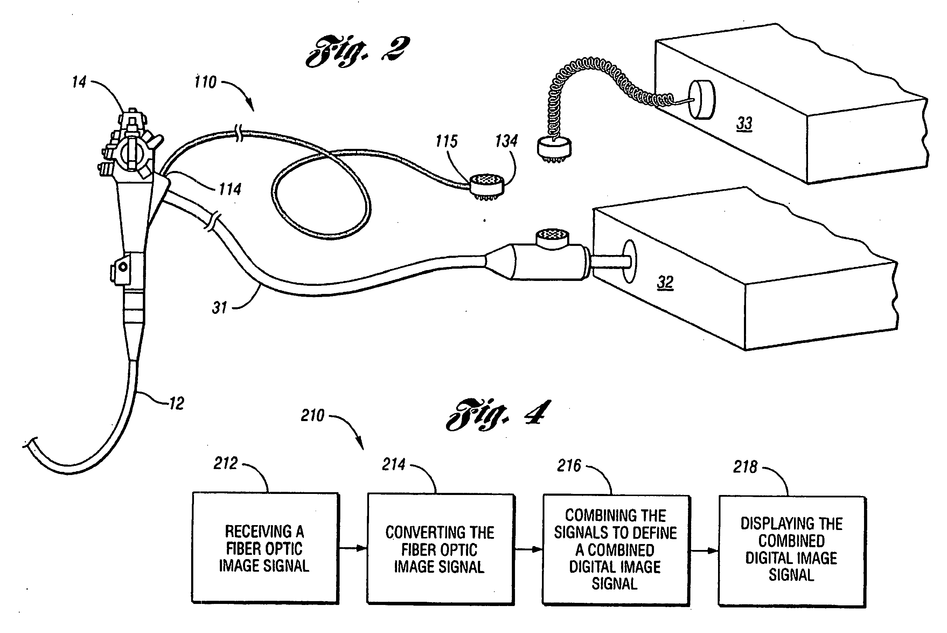

The system tower 32 is configured to receive the scope connector 18 for communication between the endoscope 11, fiberscope interfacing apparatus 110. and the system tower 32. In this embodiment, this is accomplished by attaching the interface collar 134 to the scope connector 18 as shown in Figures 2 and 3. Preferably, the scope connector 18 has an end 141 configured to connect with the system tower 32 and has a side portion 142 configured to receive the interface collar 134. As shown in Figures 2 and 3, the interface collar 134 comprises a first face 144 and a second face 145. In this embodiment, the first face 144 is a pin-out face that mates with the side portion 142 of the scope connector 18. The side portion 142 of the scope connector 18 allows for endoscope digital image signals to be transmitted to the interface collar 134. As mentioned in greater detail below, the interface collar is configured to transmit the signals to the video imaging apparatus. The transmission of digital image signals mentioned herein may be accomplished by any suitable means.

Each of U.S. patents 7,120,282; 6,985,555; 6,952,464; 6,912,266; 6,912,266; 6,975,338; 5,841,491; 5,124,789 and 6.895,077 provide backgroung information.

As shown, the video imaging apparatus 33 includes a video coupling 150 extending therefrom. In this embodiment, the video coupling 150 has a receiving pin-out face 152 that mates with the second face 145 of the interface collar 134. The interface collar 134 receives the video coupling 150 to transmit signals to the video imaging apparatus 33 for simultaneous fluoroscopic and fiber optic imaging.

In one example, images from the fiberscope are converted to fiberscope digital image signals by the interface collar. Preferably, this is accomplished by projecting the images obtained from the fiber optic core of the fiberscope onto a CCD (charged couple display) contained within the interface collar. The interface collar receives the digital endoscope image signal by interfacing the first face with the side portion of the scope connector. In this example, the fiberscope images are integrated with the endoscope.

After fluoroscopic dye is injected into a body cavity, the digital signals from the fiberscope and fluoroscope would next be combined within the interface collar so that the combined digital image signal now represents an image appearing as a "picture-in-picture" digital image. This digital signal is then outputted to the video coupling via the same pin-outs into a video display of an system tower, so that the images from both the fiberscope and fluoroscope are displayed simultaneously on the video monitor. In one embodiment, the fluoroscopic mechanism of the endoscopic system exists as a device separate from the endoscope and is only used to inject contrast medium into the body cavity so that the anatomy may be seen more clearly.

Figure 4 depicts an example of one method 210 of simultaneous fluoroscopic and fiber optic imaging during an endoscopic procedure. As shown, the method comprises receiving in box 212 a fluoroscopic digital image signal. In one example, this is accomplished by use of a fluoroscope 310 (see Figure 5) in combination with the endoscope mentioned above. As shown, the fluoroscope 310 comprises an x-ray machine 312 having an x-ray scanner, and a fluorescent screen bed (or x-ray table) 314 that is in communication with the x-ray machine 312. The video imaging apparatus 33 (mentioned above) is in communication with the x-ray machine 312 to allow for viewing of the internal organs of the body.

In this example, an intravenous (IV) line may be inserted in the patient via hand or arm while the patient is positioned on the x-ray table. Moreover, the endoscope is introduced into the patient to an area adjacent the target location, e.g., the duodenum for fluoroscopy of within the biliary tree. Fluoroscopic dye or contrast substance may be injected into the endoscope line in order to better visualize the structure being studied. That is, fluoroscopic dye may be injected into a body cavity location via the fluoroscopic value 22 of the endoscope by a fluoroscopic cannula or catheter. The body cavity location may be a location that the endoscope may otherwise not be able to access, e.g., the biliary tree. As mentioned above, once the dye is injected into the body cavity, the fluoroscopic digital image signal is generated by the fluoroscope and may be converted to real-time video as discussed herein. In one example, the x-ray scanner may be used to receive x-ray signals, convert to digital video signals, and generate fluoroscopic images of the body structure being examined or treated.

In this example, a fiber optic image signal is then received and converted in box 214 to a fiberscope digital signal by the interface collar as discussed above. Preferably, the fluoroscopic and the fiberscope digital signals are then combined in box 216 by the interface collar by any suitable means mentioned above to define a combined digital image signal. The combined digital image signal allows for real-time video display of the body cavity remote from the endoscope (e.g., an area of the biliary tree inaccessible by the endoscope) and real-time video display of the area adjacent the endoscope (e.g., the duodenum).

The method further comprises displaying in box 218 the combined digital image signal for simultaneous imaging (remote and proximal views simultaneously). This is accomplished by receiving the signal with the video coupling and transmitting the signal to the video imaging apparatus having a video monitor that displays the combined digital image in a cascading view or picture-in-picture view.

While the present invention has been described in terms of preferred embodiments, it will be understood, of course, that the invention is not limited thereto since modifications may be made to those skilled in the art; particularly in light of the foregoing teachings.

## Claims

1. An endoscopic apparatus having simultaneous fluoroscopic and fiber optic imaging features for a body cavity, the apparatus comprising:
an endoscope (11) comprising:
a control system for controlling the endoscope;
an insertable tube extending from the control system to a distal tip, the insertable tube being in communication with the control system; and
a scope medium extending to a scope connector (18) in communication with the control system;
a system tower (32) configured to receive the scope connector for communication with the control system;
a fiberscope interfacing apparatus cooperable with the endoscope, the fiberscope interfacing apparatus comprising an elongate communication medium (112) having a first end and a second end for transmitting an optical signal of an image of the body cavity, a lens (132) attached to the first end of the communication medium for receiving the optical signal from the body cavity, the lens being in communication with the communication medium and an interface collar (134) attached to the second end of the communication medium and in communication therewith and configured to connect to the scope connector (18) for receiving an endoscope digital image signal, the interface collar being configured to convert said optical signal to a fiberscope digital signal, the interface collar (134) further being configured to convert the fiberscope digital image signal and the endoscope digital signal to a combined digital image signal; and a video imaging apparatus configured to connect to the interface collar (134) for receiving the combined digital image signal for simultaneous fluoroscopic and fiber optic imaging.

2. The apparatus of claim 1 wherein the elongate communication medium comprises an optical fiber medium comprising a fiber core having a predetermined refractive index, a cladding layer disposed about the fiber core and having a lower refractive index, a resin buffer layer disposed about the cladding layer, and a jacket layer disposed about the buffer layer for transmission of the optical signal from the first end to the second end of the communication medium.

3. The apparatus of claim 1 wherein the lens is a wide-angle lens.

4. The apparatus of claim 5 wherein the fiber scope interfacing apparatus comprises a light source for emitting light through the elongate communication medium for reflecting optical signals therethrough.

## Patentansprüche

1. Endoskopisches Gerät mit simultanen fluoroskopischen und faseroptischen Bildgebungsmerkmalen für eine Körperhöhle, umfassend:
ein Endoskop (11), umfassend:
ein Kontrollsystem zur Steuerung des Endoskops;
einen sich von dem Kontrollsystem bis zu einer distalen Spitze erstreckenden einführbaren Schlauch, der mit dem Kontrollsystem in Verbindung steht; und ein sich bis zu einem Endoskop-Verbinder (18), der mit dem Kontrollsystem in Verbindung steht, erstreckendes Endoskopmedium;
ein Systemturm (32) zur Aufnahme des Endoskop-Verbinders zur Verbindung mit dem Kontrollsystem;
ein Fiberskop-Schnittstellengerät, das mit dem Endoskop zusammenarbeiten kann, wobei das Fiberskop-Schnittstellengerät ein längliches Kommunikationsmedium (112) mit einem ersten Ende und einem zweiten Ende zur Übertragung eines optischen Signals eines Bildes der Körperhöhle, eine am ersten Ende des Kommunikationsmediums befestigte Linse (132) zum Empfang des optischen Signals von der Körperhöhle, wobei die Linse mit dem Kommunikationsmedium und einem Schnittstellenkragen (134) in Verbindung steht, der am zweiten Ende des Kommunikationsmediums befestigt ist und damit in Verbindung steht und zur Verbindung mit dem Endoskop-Verbinder (18) zur Aufnahme eines digitalen Endoskopbildsignals ausgelegt ist, wobei der Schnittstellenkragen (134) zum Umwandlung des optischen Signals in ein digitales Fiberskopsignal ausgelegt ist und der Schnittstellenkragen (134) ferner zur Umwandlung des digitalen Fiberskopbildsignals und des digitalen Endoskopsignals in ein kombiniertes digitales Bildsignal ausgelegt ist; und ein Videobildgebungsgerät (33) zur Verbindung mit dem Schnittstellenkragen (134) zum Empfang des kombinierten digitalen Bildsignals zur gleichzeitigen fluoroskopischen und faseroptischen Bildgebung umfasst.

2. Gerät nach Anspruch 1, worin das längliche Kommunikationsmedium ein faseroptisches Medium umfasst, das einen Faserkern mit einem vorbestimmten Brechungsindex, einer um den Faserkern angeordneten Umhüllungsschicht mit einem geringeren Brechungsindex, einer um die Umhüllungsschicht angeordneten Harzpufferschicht und einer um die Pufferschicht angeordneten Mantelschicht zur Übertragung des optischen Signals von dem ersten Ende zum zweiten Ende des Kommunikationsmediums.

3. Gerät nach Anspruch 1, worin die Linse eine Weitwinkellinse ist.

4. Gerät nach Anspruch 5, worin das Fiberskop-Schnittstellengerät eine Lichtquelle zum Aussenden von Licht durch das längliche Kommunikationsmedium zur Reflexion von optischen Signalen durch es hindurch umfasst.

## Revendications

1. Appareil endoscopique ayant des éléments d'imagerie fluoroscopique et à fibres optiques simultanée pour une cavité corporelle, l'appareil comprenant :
un endoscope (11) comprenant :
un système de commande pour commander l'endoscope ;
un tube insérable s'étendant du système de commande à une extrémité distale, le tube insérable étant en communication avec le système de commande ; et
un milieu de scope s'étendant jusqu'à un connecteur de scope (18) en communication avec le système de commande ;
une tour de système (32) configurée pour recevoir le connecteur de scope pour communication avec le système de commande ;
un appareil d'interfaçage de fibroscope utilisable conjointement avec l'endoscope, l'appareil d'interfaçage de fibroscope comprenant un milieu de communication allongé (112) ayant une première extrémité et une deuxième extrémité pour transmettre un signal optique d'une image de la cavité corporelle, une lentille (132) fixée à la première extrémité du milieu de communication pour recevoir le signal optique provenant de la cavité corporelle, la lentille étant en communication avec le milieu de communication et une bague d'interface (134) fixée à la deuxième extrémité du milieu de communication et en communication avec celui-ci et configurée pour être raccordée au connecteur de scope (18) afin de recevoir un signal d'image numérique d'endoscope, la bague d'interface (134) étant configurée pour convertir ledit signal optique en un signal numérique de fibroscope, la bague d'interface (134) étant en outre configurée pour convertir le signal d'image numérique de fibroscope et le signal numérique d'endoscope en un signal d'image numérique combiné ; et
un appareil d'imagerie vidéo (33) configuré pour être raccordé à la bague d'interface (134) afin de recevoir le signal d'image numérique combiné pour une imagerie fluoroscopique et à fibres optiques simultanée.

2. Appareil selon la revendication 1 dans lequel le milieu de communication allongé comprend un milieu de fibres optiques comprenant un coeur de fibres ayant un indice de réfraction prédéterminé, une couche de gainage disposée autour du coeur de fibres et ayant un indice de réfraction inférieur, une couche tampon de résine disposée autour de la couche de gainage, et une couche d'enveloppe disposée autour de la couche tampon pour transmission du signal optique de la première extrémité à la deuxième extrémité du milieu de communication.

3. Appareil selon la revendication 1 dans lequel la lentille est une lentille à grand angle.

4. Appareil selon la revendication 5 dans lequel l'appareil d'interfaçage de fibroscope comprend une source de lumière pour émettre une lumière à travers le milieu de communication allongé afin de réfléchir les signaux optiques à travers celui-ci.
